# EUROPEAN PATENT APPLICATION

(11) **EP 2 979 626 A1**
(43) Date of publication of application: **03.02.2016**
(21) Application number: 14382305.2
(22) Date of filing: 01.08.2014
(51) Int. Cl.: A61B 5/00

(54) **Quantitative sensory testing devices**

(71) Applicant: Fundació Institut d'Investigació Biomèdica de Bellvitge (IDIBELL), 08907 L'Hospitalet de Llobregat (ES); Universidad Politécnica de Madrid, 28040 Madrid (ES)
(72) Inventor: Serrano Afonso, Andres Ancor, 08911 Badalona (ES); Serrano Olmedo, Jose Javier, 28702 San Sebastian de los Reyes (ES)
(74) Representative: ZBM Patents - Zea, Barlocci & Markvardsen

(57) **Abstract**

Devices for performing a plurality of Quantitative Sensory Testing (QST) tests are disclosed. In a first aspect the devices comprise a main unit; at least one extensible arm, at least a filament module and at least a thermovibrating module. In another aspect the devices comprise a main unit and at least a filament unit. The filament module comprises a rotating reel module having a plurality of compartments, wherein each of the probes is located in one of said compartments. In a third aspect the devices comprise a main unit and at least a thermovibrating module. The thermovibrating module comprises at least a piston module having a piston and a cylinder, the piston having a proximal end configured to be slidable in the cylinder. The piston may comprise heatable surface.

## Description

The present disclosure relates to examination devices and more specifically to devices for performing Quantitative Sensory Testing (QST).

### BACKGROUND ART

Pain may be defined as an unpleasant sensory and emotional experience associated with actual or potential tissue damage, or described in terms of such damage. Most pain resolves quickly but sometimes pain becomes chronic despite removal of the stimulus and apparent healing of the body. Chronic pain is defined as pain lasting more than three months. A specific subclass of chronic pain is neuropathic pain. Neuropathic pain is defined as a direct consequence of a lesion or disease affecting the somatosensory system.

Sensory testing such as bedside testing has been used by clinicians to identify and qualify neuropathic pain. With the development of standardized Quantitative Sensory Testing (QST) protocols such as was provided by the German Research Network on Neuropathic Pain (DFNS), a methodology is available to detect and quantify sensory loss and sensory gain in a standardized manner. This DFNS protocol uses 13 different mechanical and thermal stimuli (e.g. graded von Frey filaments, pin-prick devices, a pressure algometer, and quantitative thermo-testing).

This QST battery tests different sub-modalities of nerve fibers involved in the transduction of sensory information from the periphery to the spinal cord such as Aβ-fibre, Aδ-fibre and C-fibre. When present, allodynia or hyperalgesia can be quantified by measuring intensity, threshold for elicitation and duration. It has been shown that QST is sensitive for quantifying sensory abnormalities on an individual patient level. Subsequently, it may be presumed that QST applied in large patient samples might allow to discriminate distinct responders to the different stimuli on a group level.

The 13 QST parameters are:

Warm Detection Threshold (WDT), Heat Pain Threshold (HPT), Cold Detection Threshold (CDT), Cold Pain Threshold (CPT), Thermal Sensory Limen (TSL), Paradoxical Heat Sensation (PHS), Mechanical Detection Threshold (MDT), Mechanical Pain Threshold (MPT), Mechanical Pain Sensitivity (MPS), Dynamic Mechanical Allodynia (DMA or ALL), Wind Up Ratio (WUR), Vibration Disappearance Threshold (VDT) and Pressure Pain Threshold (PPT).

All nerve fiber functionality tests are applied to the skin with exception of PPT (deep tissue C-fiber/Aδ-fiber test).

The thermal stimuli, are provided by a number of devices such as a thermotest machine, a CHEPS, a Pathway MEDOC (TSA 2001 - II (MEDOC, Israel), or an MSA (Somedic , Sweden). They are typically performed using a probe of about 9.0 cm2 for the TSA or 12.5 cm2 for the MSA through a transducer. Depending on which parts of the body the test is to be performed it is sometimes difficult to perform the test with these machines. Additionally there are areas where, because of the size of the probe, it may not be easy to differentiate between areas corresponding to different nerve units.

The mechanical stimuli are: MDT, MPT, MPS, ALL or DMA, WUR, VDT and PPT. Every one of those 7 stimuli is currently explored by a qualified professional (usually a doctor, nurse or other qualified technician) following a systematic attempt so that the stimuli are as objective and repetitive as possible.

The MDT is performed using von Frey filaments, with a thickness that increases logarithmically. These filaments (typically 16 in total) are positioned on the skin (on a desired examination point) until they bend, and the subject must respond if they notice or not the touch of the filament. When bent, the filaments exert a pressure of between 0.25mN and 512mn. To attempt the test as objectively as possible, the following procedure is followed: Using a metronome, the filament is changed every second with the next thicker filament. The thickness increases until the patient perceives the touch. Thickness increases two or three times more before the descent in thickness begins. Then the same operation is performed reversely. This is repeated until 5 measurements are acquired where the patients indicates if he perceives or not the filament. Then the average of these measurements is calculated. That is the MDT value.

The MPT is calculated using a blunt needle of 0.2 mm in diameter. In the same manner as with the von Frey filaments 7 needles are used with a load weight (using a cylinder and a spring) that they exert equivalent forces progressively increasing from 8, 16 , 32, 64 , 128, 256 , up to 512 mN . The MPT is calculated with the same routine as the TDM, i.e. every 2 seconds one needle with increasing weight is used until the patient notices pain. Then it increases 1-2 times more and decreases again until the patient stops noticing. The process is repeated until 5 measurements are taken that may give a average. The average shall be compared to normal values, with their standard deviation (SD) and with the contralateral side (where the same process is performed).

The MPS is evaluated with the same set of 7 needles of pinprick. Subjects are asked to assign a value from 0-100 ('0 ' indicating no pain and '100' indicating the highest imaginable pain) to each of the 7 stimuli. The test is designed for hyperalgesia.

The ALL or DMA test is explored as part of the MPS test using a set of three delicate tactile stimuli as stimulus of innocuous movement: a tuft of cotton exerting a force of 3 mN, a cotton tip attached to an elastic strip which exerts a force of 100 mN and a standardized brush (like the one used in the Somedic device) exerting a force of 200-400 mN. Tactile stimuli are applied in one stroke of about 2 cm in length on the skin. These stimuli are inserted into the MPS protocol evenly between pinprick stimuli.

A total of 50 stimuli, 15 pins and 35 touches at each site are performed wherein the subject assigns numerical pain ratings for each stimulus. These stimuli are given in sets of 10 (five sets for each examination site) and each run consists of a pseudorandom sequence of three different tactile stimuli and pinprick 7 (shot). All stimuli are performed with an interval of 10s - well below the critical frequency to display a wind-up phenomenon. MPS is calculated as the geometric mean of all numerical ratings to the pinprick stimuli. The ALL value is calculated as the geometric mean of all the numerical ratings for all three different types of tactile stimulators.

The WUR is determined by a single stimulus with a pinprick needle similar to the one used for MPT and MPS (128 mN pinprick , when applied on the face of the subject and 256 mN pinprick ,when applied on the rest of the body) . This is compared with a series of 10 repetitive stimuli of the same physical intensity (1/s within an area of 1 cm²). The subject must indicate a single stimulus number (0-100) and the estimated mean of the subsequent series. The entire process is repeated 5 times. The WUR is calculated as the ratio of the average of the 5 series divided by the average of the single stimuli. The Wind-up phenomenon is the increased frequency - dependent excitability of neurons in the spinal cord, reaching a plateau after five successive stimuli.

The VDT is calculated as follows: A medical tuning fork vibrates at 64Hz and is placed in a bone-contacting surface of the subject. The subject should indicate when he stops noticing the vibration. Therefore it is a reverse test when compared to the previous tests, and it can only be performed using the method of limits. The time between the placing of the tuning fork and the indication of the subject is timed. The process is repeated 5 times to obtain an average. The vibrating of the tuning fork, the placing, and the timing are tasks performed by the practitioner taking the measurement. A vibrameter (such as the neurothesiometer by Horwell) exists that allows modulating the frequency of vibration by a piston with a standard surface and perform a detection of a threshold of ascending vibrations (through limits and/or levels).

The PPT is calculated as follows: The practitioner that performs the examination places an algometer (a small device with a piston of a standardized measure connected to a pressure gauge in the area to explore. Every second (thanks to the metronome) the pressure increases from 50 kPa with 50 kPa increments, until the subject notices pain. The process is repeated 5 times to obtain an average. Digital algometers (such as the algometer by Somedic) exist enabling testing by the method of the levels, increasing pressure at a constant rate of 50 kPa/sec (the change of pressure is always performed manually by the practitioner that performs the examination), which allows record the exact pressure that is allowed to perform the pressure at which the subject says notice pain.

Using existing systems, the whole process of performing the complete QST test requires over an hour of examination. It requires at least a thermotest machine, von Frey filaments, pinprick needles (which are typically custom - made, implying differences in calibration and measurement of the needles used in different centers and countries), an algometer, a tuning fork and a metronome. It requires that the examination area is as extensive as possible to allow placing of the thermotest probe. It also requires that the practitioner that performs the examination is experienced, and qualified to perform such practices. The examination is therefore "practitioner-dependent." A different practitioner could imply that the results are not comparable for the same patient over time.

There is a need for QST devices that at least partially solve the aforementioned problems.

### SUMMARY OF THE INVENTION

In a first aspect, a device for performing a plurality of Quantitative Sensory Testing (QST) tests is disclosed. The device may comprise a main unit, at least one extensible arm, at least one filament module and at least one thermovibrating module. The at least one extensible arm may have a proximal end coupled to the main unit. The at least one filament module may be coupled to a first distal end of the at least one arm. The filament module may comprise a plurality of von Frey type filament probes and at least one pinprick type filament probe. The at least one thermovibrating module may be coupled to a second distal end of the at least one arm.

The proposed device allows the QST tests to be performed in an objective and reproducible way. It allows such tests to be comparable for a single patient over time or between patients. The extensible arm allows for the collocation of the test modules, either the filament or the thermovibrating module, to any part of the patient's body. Therefore all tests may be performed while the patient is unmovable, e.g. lying on a bed. Furthermore, as the tests are integrated, they can all be performed in a controlled time period. For example, they may be performed successively without requiring for the patient to change position or disposition. As the test is integrated, the reaction time of the patient may be reduced. A reduced testing time increases the objectivity of QST tests as they are psychophysical tests depending on the disposition of the patient.

The filament module may perform one or more of the following QST tests: MDT, MPT, MPS, WUR and ALL. The thermovibrating module may perform one or more of the following QST tests: thermal (WDT, HPT, CDT, FPT, WLT, and CLT), vibratory (VDT) and pressure (PPT).

The main unit may comprise a motherboard with software and connections for each test module. The software may allow the registration and integration of data from all the tests in one spreadsheet and/or document. It may allow the implementation of different examination programs and change any limits or level of each testing method type (if the test so allows).

In some embodiments the at least one extensible arm may comprise a first extensible arm and a second extensible arm. The first arm may be coupled to the filament module and the second arm may be coupled to the thermovibrating module. This allows for simultaneously performing each test, e.g. to a different patient, or to quickly perform the first test followed by the second test in, e.g. a different area of the body of the patient.

In some embodiments each arm may comprise a plurality of joints configured to attribute a plurality of degrees of freedom of movement to the filament and/or the thermovibrating modules. This allows placement of each of the filament and thermovibrating modules at any part of the body of the patient without the need to reposition the body or the main unit. It also allows the tests to be performed at any part of the patient's body without the need to move the device. The joints may for example be ball and socket type joints or bearing joints that permit motion around an indefinite number of axes between connected ball and socket components to together provide each arm with sufficient flexibility to assume any position with respect to the main unit. The friction coefficient between the ball and the socket or of the bearing may be such that the position of each joint, that is the relative position of the ball and the socket, may be maintained once assumed. Each joint may further comprise openings so that any wiring or fluid piping required for the tests may pass through the joints.

In some embodiments each joint may be independently controllable by the main unit. A user may guide the joints from the main unit and position the modules where the tests are to be performed. Alternatively or complementarily the joints may be manually controlled so that the modules are brought in line with the part of the patient's body where the tests are desired to be performed.

In some embodiments the filament module may comprise a rotating reel module having a plurality of compartments. Each of the probes may be located in one of said compartments. The compartments may comprise all the von Frey filaments in order of size. For the MDT test, the rotating reel module may rotate the reel so that a different von Frey filament is used, e.g. every second, exerting the required pressure until it curves. The von Frey filaments may be used in successive order of thickness. Once the patient indicates that he has sensed the filament, then the process may continue for two or three thicker filaments in order to confirm the test perception. Accordingly, the test may be reversed and performed in decreasing order of thickness and continue until the patient no longer feels the filaments. To confirm the result, the test may continue for two or three filaments more after no perception is indicated. This way, the test exploration that is known in the art may be performed in a mechanized, synchronized, repetitive and reproducible manner. The rotating reel module may further comprise a mechanism, e.g. a motor, for rotating the reel.

One of the filaments may be a friction type brush or a conduit for dispensing air to measure soft tactile sensitivity with different pressures in a controlled manner. Thus, the MPS and ALL tests may be performed.

The pinprick probe may be placed in a separate compartment of the reel module. The pinprick probe may be disposable and replaceable to avoid contamination or spread of infections. The MPT test may be performed by pushing the pinprick probe (or needle) against the patient's body and withdrawing the needle at 2 second intervals. Each time the pressure force may be increased by a corresponding number of miliNewtons (mN) with the aim to surpass the 512 mN.

In some embodiments the rotating reel module may further comprise an opening. Rotation of the reel may align one of the probes with said opening thereby allowing output of the aligned probe. This way, the same position of the skin may be used for the stimulations. Furthermore, the same position of the skin may be used for the MDT and the MPT test.

With the same filament module it is also possible to perform the WUR test. It may be performed with the pinprick needle. In this case, the pressure is predetermined according to the point of examination and it may be performed according to the QST specifications in a mechanized manner.

In some embodiments the filament module may further comprise a probe actuator. The probe actuator may engage with a selected probe to perform the desired test. The probe actuator may comprise a piston module having a piston and a cylinder. The piston may be configured to be displaceable towards the rotating reel module. The piston may comprise a proximal end configured to be slidable in the piston and a distal end configured to be coupled to a coupling element of a probe. The distal end of the piston may comprise a fastening element and the coupling element of the probe may comprise a corresponding fastening element. When the piston is aligned with the opening of the reel, then the compartment containing the desired test filament is present in the opening. The actuator may then displace the piston to engage with the filament to perform the test. In case the test is a von Frey test, the piston may be required to travel a predetermined distance until the filament bends. In case it is a pinprick test, the piston must exert a precise pressure, among a plurality of possible ones, which needs to be exerted in a predetermined order.

In some embodiments the filament module may further comprise a fastening element for coupling to said at least one arm or to said probe actuator. Fastening and unfastening of the reel module allows for easy replacement of filaments and servicing of the module without requiring the whole device to be inoperable. For example, while a filament module is being repaired or refilled, another filament module may be fastened to the same device.

In some embodiments the thermovibrating unit may comprise a piston module having a piston and a cylinder. The probe may comprise a proximal end configured to be slideable in the cylinder. The distal end may comprise a heatable surface. The piston module may be used for any thermal (WDT, HPT, CDT, CPT, TSL and PHS tests) or vibration (PPT or VDT) tests that form part of the QST test.

For vibration tests, the piston may exert a controlled pressure of increments of 50kPa/sec. This method allows for the PPT examination with the limits method (continued rise in time) or with the levels method with pressure rises from 50kPa in a rate of 50 kPa/s.

It also allows a vibration examination using the piston as a continuous and repetitive percussion piston. This method accomplishes the VDT examination. The vibration may start at 64 Hz and may decrease at a rate of "z" Hz/sec (thus reproducing the effect of the medical tuning fork) for the method of limits. For the method of levels, the percussion can be performed in an increasing and/or decreasing manner, thus reproducing the MDT test for the vibratory field.

Now the heatable surface may be flat. The heatable surface may be heated when a thermal test is to be performed. Otherwise it may simply contribute to a vibration test with its flat surface.

The heatable surface may be covered by a material that allows swift heating and cooling in a controlled manner and rate. The correct progress of the temperature may be controlled by sensors. Heating may be performed by a high amplitude DC current. Cooling may be performed by an abrupt evaporation of a refrigerating liquid.

The thermovibrating module may also further comprise a fastening element for coupling to said at least one arm. That way, it may be easily replaced or serviced without the need to interrupt the use of the device as it may be replaced by another module in the meantime.

In some embodiments the device may further comprise at least one positioning module coupled at least to the filament or to the thermovibrating module. The positioning module may manually or automatically precisely place the corresponding module at a desired area above a desired area of the body where the test is to be performed. This allows for a precise reproducibility of any test.

In some embodiments the positioning module may comprise an opening allowing the probe to pass through the positioning module and contact the part of the body at a position indicated by said positioning module. Therefore the module may be integrated in the device and the selected position on the patient's body may be validated at any moment.

In some embodiments the positioning module may comprise a light emitting module (such as laser module or LED module). The light emitting module may guarantee precision of the position of the test. The light emitting module may comprise a first light pointer, e.g. a kaser pointer, emitting a first light beam and a second light pointer emitting a second light beam. The pointer beams may be configured so that when they coincide at the contact position, the test module may be considered optimally positioned. This may be performed manually or automatically. For example, the user may guide the joints in response to the light beams until the two beams coincide on the patient's body at the desired position. The user of the device may visually verify with the use of the positioning module that the test modules may be at the appropriate distance from the body and that the surface of the filament or of the thermovibrating module may be parallel to a tangential surface of the part of the body where the test is to be performed. Alternatively, an ultrasound module may be used.

In some embodiments the positioning module may further comprise one or more piezoelectric transducers configured to obtain distance and direction information from the surface of the part of the patient's body. This guarantees that the modules remain at the desired position throughout the tests.

In a second aspect, another device for performing QST tests is disclosed. The device according to the second aspect may comprise a main unit and at least a filament module. The filament module may comprise a plurality of von Frey type filament probes and at least one pinprick type filament probe. The filament module may comprise a rotating reel module having a plurality of compartments. Each of the probes may be located in one of said compartments. Devices according to the second aspect have the advantage of performing filament tests in a precise and reproducible manner. The integration of the von Frey type filaments with the pinprick type filaments in the same reel module allows for the various mechanical tests to be performed in the same position on the patient's body thus increasing the speed, accuracy and reproducibility of the tests.

In some embodiments of the second aspect, the rotating reel module may further comprise an opening wherein rotation of the reel aligns one of the probes with said opening thereby allowing output of the aligned probe. The rotating reel module may further comprise a mechanism for rotating the reel. The filament module may further comprise a probe actuator. The probe actuator may comprise a piston module having a piston and a cylinder. The piston may be configured to be displaceable towards the rotating reel module. The piston may comprise a proximal end configured to be slidable in the cylinder and a distal end configured to be coupled to a coupling element of an aligned probe. The distal end may comprise a fastening element and the coupling element may comprise a corresponding fastening element. The function of the probe actuator may be similar to the one discussed with reference to embodiments according to the first aspect disclosed herein.

In some embodiments according to the second aspect the device may further comprise at least one arm, having a proximal end coupled to the main unit and a distal end coupled to the filament module. The arm may be an extensible arm

In some embodiments according to the second aspect the device may further comprise at least a thermovibrating module coupled to a second distal end of the at least one arm. The at least one arm may comprise a first arm and a second arm. The first arm may be coupled to the filament module and the second arm may be coupled to the thermovibrating module.

In embodiments according to the second aspect each arm may comprise a plurality of joints, configured to attribute a plurality of degrees of freedom of movement to the filament and/or the thermovibrating modules. Each joint may be independently controllable by the main unit or manually controllable by the user. The thermovibrating unit may comprise a piston module having a piston and a cylinder. The piston may have a proximal end configured to be slideable in the cylinder. A distal end may comprise a heatable flat surface.

In embodiments according to the second aspect the device may further comprise at least one positioning module coupled at least to the filament or to the thermovibrating module. In some implementations each of the filament and thermovibrating modules may be coupled to a positioning module. The positioning module(s) may be similar to the positioning modules described with reference to the first aspect disclosed herein.

In a third aspect, yet another device for performing a plurality of Quantitative Sensory Testing (QST) tests is disclosed. The device may comprise a main unit and at least a thermovibrating module. The thermovibrating module may be coupled to the main unit. The thermovibrating unit may comprise a piston module having a piston and a cylinder. The piston may have a proximal end configured to be slidable in the cylinder. The distal end of the piston may be heatable. By making the thermovibrating module heatable it is possible to integrate vibration QST tests with thermal QST tests. The tests may then be performed in a controlled and reproducible way. Furthermore, the intervention of any person that performs the test may be minimized and the overall time of the test may be reduced. The thermovibrating module may be similar to the thermovibrating modules described herein with reference to embodiments of the first and second aspects.

Embodiments of the third aspect may further comprise at least one arm similar to embodiments described with reference to the first and second aspects disclosed herein. Accordingly, the device may further comprise at least a filament module similar to embodiments described with reference to the first aspect and second aspects disclosed herein. Furthermore, the embodiments of the device according to the third aspect may further comprise at least one positioning module similar to embodiments described with reference to the first aspect.

In embodiments according to any aspect disclosed herein the main unit may comprise a controller configured to control one or more of the filament module, the thermovibrating module and the at least one arm. The main unit may further comprise a user interface module. The user interface module may comprise a display. The display may be a touch sensitive display. The user interface module may also comprise a keyboard. Alternatively the keyboard may be implemented in software and visualized in the touch sensitive display. In embodiments according to any aspect disclosed herein, the device may further comprise an input device configured to be used by the patient to indicate a perception of a test. The input device may comprise at least one button and the user may indicate the perception of a test by pressing the button. The presence of the input device may make the patient more comfortable with the test and may minimize the reaction time as the patient doesn't need to speak. The input device may comprise at least one ergonomic joystick. This may allow the patient to hold the input device while in a reclined position and use his thumb to indicate a test perception.

In embodiments according to any aspect disclosed herein the device may further comprise an audio and/or visual stimulating device to alert the patent about the beginning of a test. Therefore the patient may be more concentrated during the test, thus the results may be more accurate. Furthermore, the response of the patient may be faster, thus reducing the overall time of the test.

In embodiments according to any aspect disclosed herein, the main unit may further comprise a test simulation module. The test simulation module may be configured to perform erroneous tests or to actuate the audio and/or visual stimulating device without performing a test. In that way, the device may be tested and also the patient may be tested as to his ability to detect erroneous or false stimulants.

In yet another aspect, a controller is disclosed. The controller may be configured to control a device according to embodiments disclosed herein. The controller may comprise a memory and a processor. The memory may store computer program instructions executable by the processor. Said instructions may comprise functionality to control a device for performing a QST test according to any of the embodiments disclosed herein. For example, the controller may control the arm so that it assumes a desired position. Furthermore, the controller may select the test to be performed and instruct the filament module or the thermovibrating module accordingly.

In yet another aspect, a computer program product is disclosed. The computer product may comprise instructions to provoke that a controller implements a method of controlling a device for performing QST tests according to embodiments disclosed herein. The computer program product may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the processes according to the invention. The carrier may be any entity or device capable of carrying the computer program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

### BRIEF DESCRIPTION OF THE DRAWINGS

Particular embodiments of the present invention will be described in the following by way of non-limiting examples, with reference to the appended drawings, in which:
Figure 1 is a schematic illustration of a device for performing QST tests according to an example;
Fig. 2a and 2b illustrate a front and back view, respectively, of a filament module according to an example;
Figure 3 illustrates a side view of a filament module according to an example;
Fig. 4a and 4b illustrate a front and back view, respectively of an actuator module according to an example;
Figure 5 illustrates an actuator module according to an example;
Fig. 6a and 6b illustrate a front and back view, respectively, of a thermovibrating module according to an example;
Fig. 7 illustrates a thermovibrating module according to an example;
Fig. 8a, 8b and 8c illustrates a front, side and back view, respectively, of a positioning module according to an example.

### DETAILED DESCRIPTION OF EMBODIMENTS

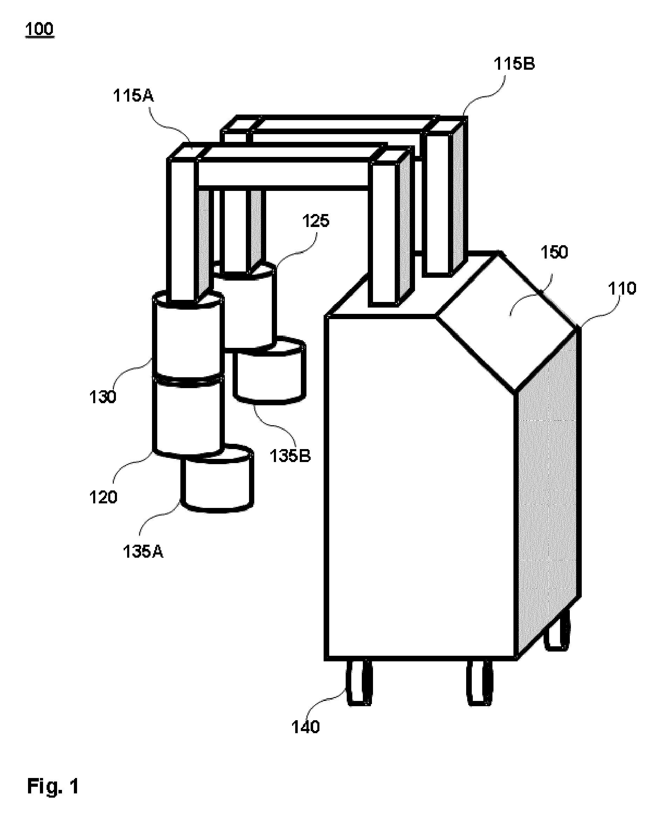

Fig. 1 is a schematic illustration of a device for performing QST tests according to an example. Device 100 comprises a main unit 110, a pair of extensible arms 115A, 115B and a plurality of test modules. The main unit may have a user interface 150. The user interface may comprise a display, such as a touch sensitive display and a keyboard. Alternatively the keyboard may form part of the touch sensitive display. The main unit 110 may comprise a controller (not shown) for controlling the extensible arms and/or the test modules. In other implementations the controller may be external to the main unit. For example the controller may be a computer connected to the main unit and configured to execute instructions to perform the functions of controlling the extensible arms and/or the test modules. The main unit may further comprise a set of wheels 140 to facilitate transportation of the device. The extensible arms 115A, 115B may be connected to the main unit. Each extensible arm may have a fixed part coupled to the main unit at a proximal end of the arm and an extensible part. The extensible part may comprise joints. Each joint may connect articulating parts of the extensible arm. The joints may provide one or more degrees of freedom to the articulating parts. The distal end of each arm may be coupled to a test module. The test modules may thus be extended so that they may reach any part of a patient's body to perform a test. First extensible arm 115A is shown coupled to actuator 130 of filament module 120. Filament module 120 is coupled to a first positioning module 135A. The order and number of modules is merely illustrative and may serve only as an example of a possible configuration. One skilled in the art may appreciate that the modules may be arranged in a different order or configuration. Furthermore, one or more of the modules may be integrated in one module. Furthermore, a module may actually be arranged in a plurality of interconnected sub-modules. The second extensible arm 115B is shown coupled to thermovibrating module 125 at its distal end. Thermovibrating module 125 may be further coupled to a second positioning module 135B.

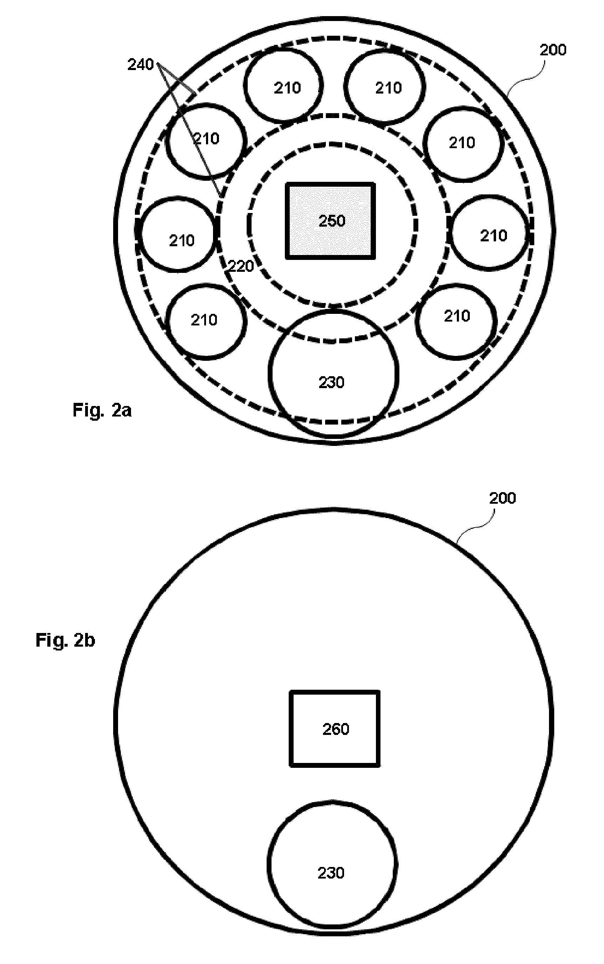

Fig. 2a and 2b illustrate a front and back view, respectively, of a filament module according to an example. The filament module 200 comprises a plurality of compartments 210. The number of compartments illustrated in Fig. 2 is merely indicative. The filament module 200 may be configured to accommodate any number of compartments required for performing the mechanical tests of the QST tests. Each compartment 210 may be configured to receive a von Frey filament or a pinprick needle. The filament module may also comprise an opening 230. The filament module 200 may comprise a rotatable reel 240 and may comprise a revolving mechanism 220 so that each of the compartments 210 may become aligned with the opening 230 when a particular filament is desired for a test. The filament module may further comprise a first fastening element 250 for attaching to a positioning module and a second fastening element 260 for attaching to an extensible arm or to an actuator module.

When a mechanical test is selected, the controller may instruct the rotating reel to rotate so that the compartment containing the first desired filament assumes its position in the opening 230. Then, according to the selected test protocol, the rotating reel 240 will rotate at predetermined intervals, so that another compartment assumes position in the opening.

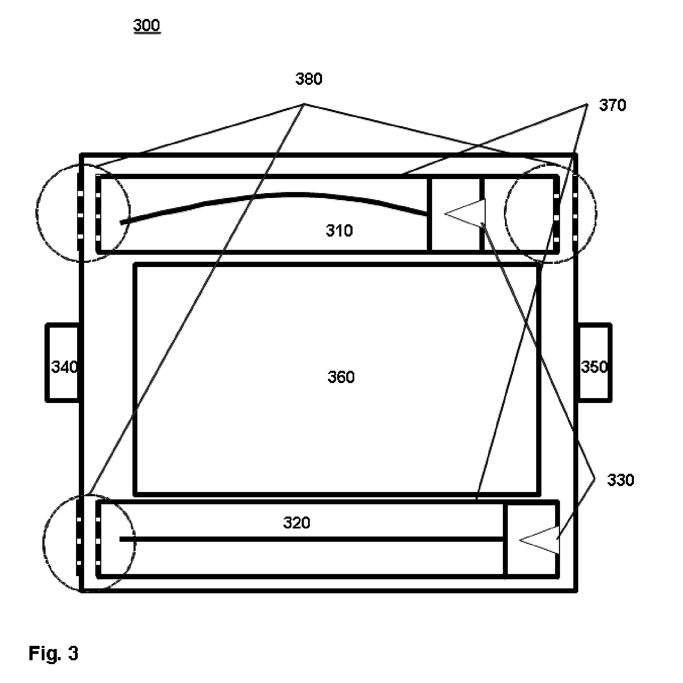

Fig. 3 illustrates a side view of a filament module according to an example. A von Frey filament 310 is shown in a bent position inside a filament compartment 370. Another filament compartment 370 hosts a pinprick needle 320. The von Frey filament and the pinprick needle are each connected to a coupling element 330. The coupling elements 330 may be connectable to a mating element (not shown) on an actuator module. An opening 380 on the side of the coupling elements allows the mating element to enter the compartment and connect with the coupling element 330. When a coupling element is connected to a mating element, the compartment of the probe (filament or needle) may be aligned with the opening of the filament module and may be displaced towards the opening 380 of its compartment. The compartments 370 may form part of the rotating reel. The rotating reel may be actuated by an actuating mechanism 360. The filament module may further comprise a fastening element 340 for attaching to a positioning module. Finally, the filament module may comprise a fastening element 350 for attaching to the actuator module.

When a desired test is selected, the compartment containing the first desired filament is aligned with the opening 380. Then a mating element engages with the coupling element of the first desired filament and the filament is displaced towards the body of the patient. Then the mating element is withdrawn and the corresponding filament assumes position in its compartment. The reel may then rotate to provide another compartment with filament in the opening.

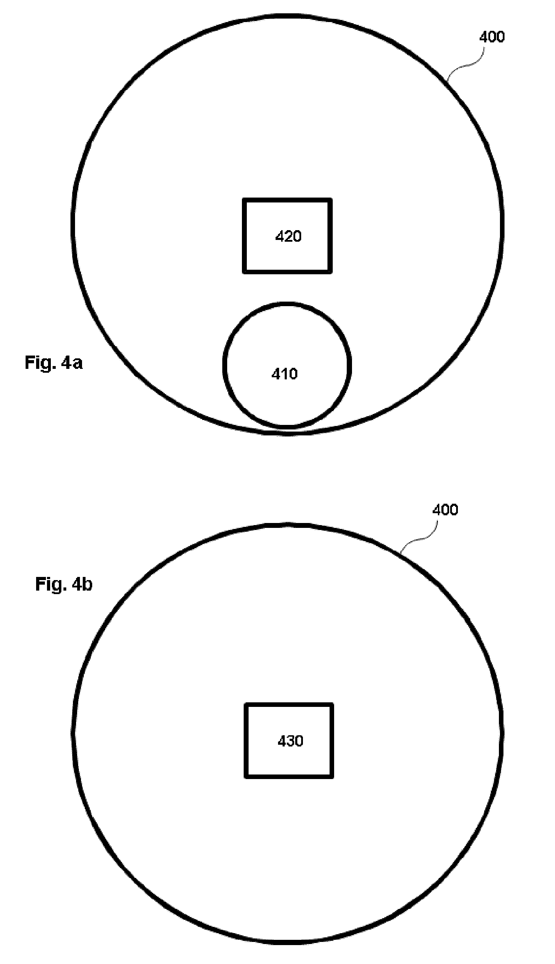

Fig. 4a and 4b illustrate a front and back view, respectively of an actuator module according to an example. Actuator module 400 comprises an opening 410 for allowing a mating element, such as a piston rod, to move transversely and couple to a coupling element of a probe. The actuator module may further comprise a first fastening element 420 for attaching to a filament module and a second fastening element 430 for attaching to an arm, such as an extensible arm.

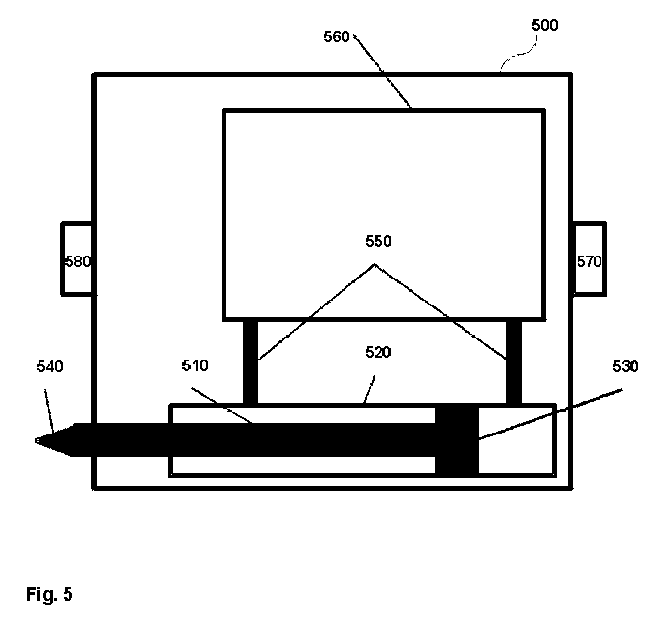

Fig. 5 illustrates an actuator module according to an example. The actuator module 500 comprises a piston module having a piston cylinder 520 and a mating element 510. The mating element 510 may have a proximal end 530 slideable inside the piston cylinder 520. The distal end 540 of the probe 510 may be configured as a fastening element, e.g. a pointed end. It may then mate with a corresponding fastening element, e.g. a coupling element, such as the coupling elements described with reference to Fig. 3. The actuator module 500 may further comprise a fluid discharge mechanism 560 and a pair of fluid conductors coupled to the piston cylinder for conducting the fluid. A first fastening element 570 may be coupled to an arm and a second fastening element 580 may be coupled to a filament module.

In use, the controller may identify a desired filament ready to perform a test aligned with the opening of the rotating reel module. Then the controller may instruct the actuator module to displace the piston 510 towards the rotating reel module so that the mating element 540 engages with the coupling element of the corresponding filament. When the patient indicates (e.g. with a joystick) that he has perceived the filament, then the piston 510 may disengage from the filament and the process may continue with the next filament or with another test.

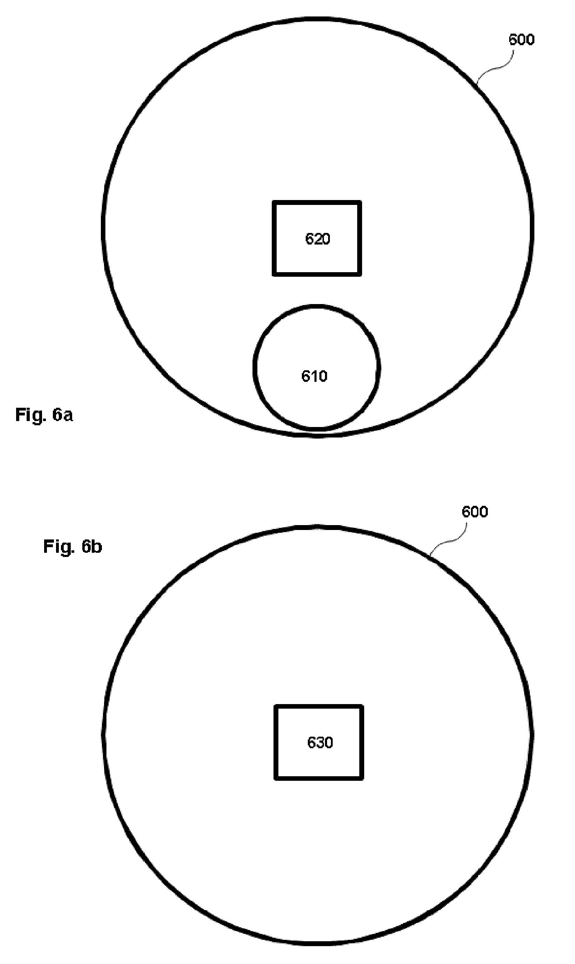

Fig. 6a and 6b illustrate a front and back view, respectively, of a thermovibrating module according to an example. Thermovibrating module 600 comprises an opening 610 for allowing a vibrating element, such as a piston, to vibrate transversely and perform the vibrating action required by vibrating QST tests. Furthermore, the vibrating element may be heatable in order to provide the heat characteristics required by thermal QST tests. The thermovibrating module may further comprise a first fastening element 620 for attaching to a positioning module and a second fastening element 630 for attaching to an arm, such as an extensible arm.

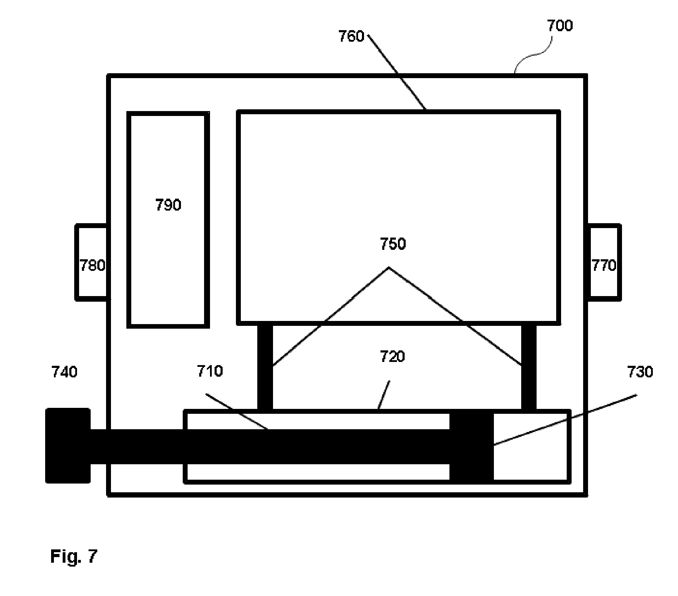

Fig. 7 illustrates a thermovibrating module according to an example. The thermovibrating module 700 comprises a piston module having a piston cylinder 720 and a piston 710. The piston 710 may have a proximal end 730 slideable inside the piston cylinder 720. The distal end 740 of the piston 710 may be configured as a flat surface and it may be heatable. The thermovibrating module 700 may further comprise a fluid discharge mechanism 760 and a pair of fluid conductors 750 coupled to the piston cylinder 720 for conducting the fluid. A first fastening element 770 may be coupled to an arm and a second fastening element 780 may be coupled to a positioning module.

In use, the controller may identify a desired thermal or vibrating QST test to be performed. In case of a vibrating test, the controller may instruct the thermovibrating module to vibrate the piston 710 with a corresponding vibration rate to perform the test. In case of a thermal test, the controller may instruct the thermovibrating module to heat or cool the heatable surface 740 according to the test's protocol. The heatable surface 740 may comprise a resistor that may be abruptly heated with DC current and the thermovibrating module may further comprise a means for providing a coolant. For example the thermovibrating module may comprise a means for evaporating a liquid refrigerant. When the patient indicates (e.g. with a joystick) that he has perceived the presence or absence of a vibration or heat, then the piston 710 may stop and the process may continue with another test.

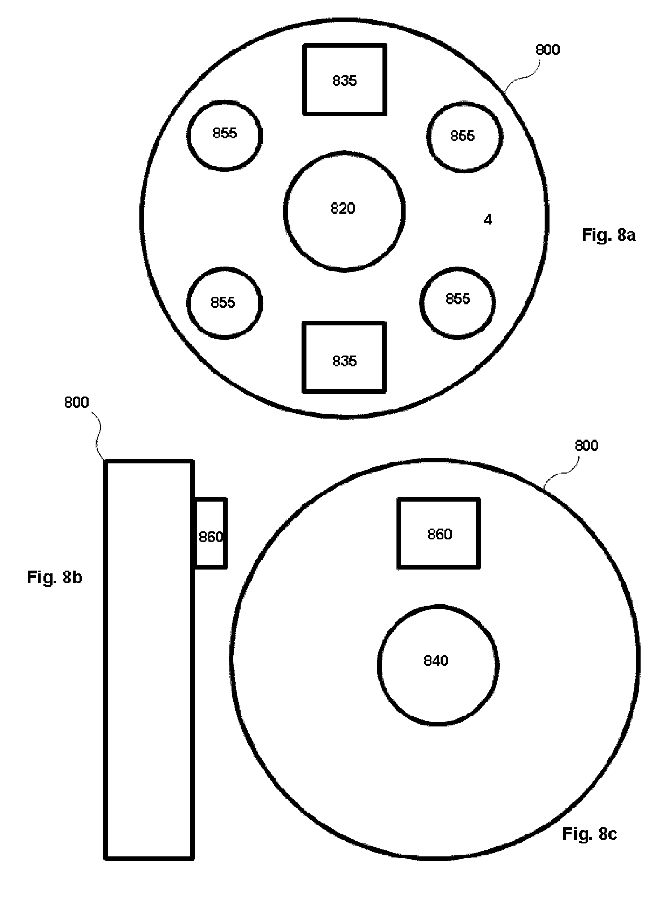

Fig. 8a, 8b and 8c illustrates a front, side and back view, respectively, of a positioning module according to an example. Positioning module 800 may comprise a pair of light emitting units, e.g. laser units 835. Each laser unit 835 may be configured to emit a laser beam. The laser beams may coincide on a position where the test is to be performed. This facilitates the positioning of the modules with respect to the patient's body. Therefore, any test may be reproduced with the same characteristics for the same patient or between patients. The positioning module 835 may further comprise an opening 820. The opening may be aligned with the opening of the filament module or the thermovibrating module so that any filament or piston may pass through the positioning module during the respective test. The positioning module may further comprise a plurality of piezoelectric transducers configured to obtain distance and direction information from the surface of the part of the body where the test is performed. This information may be used during the test so that any movement of the body of the patient may be recorded and any test be repeated if required. The positioning module may further comprise a fastening element 860 configured to couple with the corresponding fastening elements 580 and 780 of the filament module or of the thermovibrating module.

In use, the controller or the user of the device may direct the arms towards the position where a desired QST test is to be performed and position the corresponding positioning module in such a way that the two light beams coincide at the desired test position. When the module is in place, then the test may begin.

For reasons of completeness, various aspects of the present disclosure are set out in the following numbered clauses:
Clause 1. A device for performing a plurality of Quantitative Sensory Testing (QST) tests, the device comprising:
   a main unit;
   at least one extensible arm, having a proximal end coupled to the main unit;
   at least one filament module coupled to a first distal end of the at least one arm, said filament module comprising a plurality of von Frey type filament probes and at least one pinprick type filament probe;
   at least one thermovibrating module coupled to a second distal end of the at least one arm.
Clause 2. The device according to clause 1, wherein the at least one extensible arm comprises a first extensible arm and a second extensible arm, wherein the first extensible arm is coupled to the filament module and the second extensible arm is coupled to the thermovibrating module.
Clause 3. The device according to clause 1 or 2, wherein each arm comprises a plurality of joints, configured to attribute a plurality of degrees of freedom of movement to the filament and/or to the thermovibrating modules thereby allowing placement of each of the filament and thermovibrating modules at any part of the body without the need to reposition the body.
Clause 4. The device according to clause 3, wherein each joint is independently controllable by the main unit.
Clause 5. The device according to any of the above clauses wherein the filament module comprises a rotating reel module having a plurality of compartments, wherein each of the probes is located in one of said compartments.
Clause 6. The device according to clause 5, wherein the rotating reel module further comprises an opening wherein rotation of the reel aligns one of the probes with said opening thereby allowing output of the aligned probe.
Clause 7. The device according to clauses 5 or 6, wherein the rotating reel module further comprises a mechanism for rotating the reel.
Clause 8. The device according to any of clauses 5 to 7, wherein the filament module further comprises a fastening element for coupling to said at least one arm.
Clause 9. The device according to any of clauses 5 to 8, the filament module further comprising a probe actuator.
Clause 10. The device according to any of the previous clauses, wherein the probe actuator comprises a piston module having a piston and a cylinder, the piston configured to be displaceable towards the rotating reel module.
Clause 11. The device according to clause 10, wherein the piston comprises a proximal end configured to be slidable in the cylinder and a distal end configured to be coupled to a coupling element of a probe.
Clause 12. The device according to clause 11, wherein the distal end comprises a fastening element and the coupling element comprises a corresponding fastening element.
Clause 13. The device according to any of previous clauses, wherein the thermovibrating module comprises a piston module having a piston and a cylinder, the piston having a proximal end configured to be slidable in the cylinder.
Clause 14. The device according to clause 13, wherein a distal end of the piston comprises a heatable surface.
Clause 15. The device according to any of clauses 13 to 14, wherein the thermovibrating module further comprises a fastening element for coupling to said at least one arm.
Clause 16. The device according to any of the previous clauses, further comprising at least one positioning module coupled at least to the filament or thermovibrating module.
Clause 17. The device according to clause 16, wherein the positioning module comprises an opening allowing said probe to pass through the positioning module and contact the part of the body at a position indicated by said positioning module.
Clause 18. The device according to clause 16 or 17, wherein the positioning module comprises a light emitting module.
Clause 19. The device according to clause 17, wherein the light emitting module comprises a first light pointer emitting a first light beam and a second light pointer emitting a second light beam, wherein the pointer beams are configured to coincide at the contact position.
Clause 20. The device according to any of clauses 16 to 19, wherein the positioning module further comprises one or more piezoelectric transducers configured to obtain distance and direction information from the surface of the part of the body.
Clause 21. A device for performing a plurality of Quantitative Sensory Testing (QST) tests, the device comprising:
   a main unit;
   at least a filament module coupled to a first distal end of the at least one arm, said filament module comprising a plurality of von Frey type filament probes and at least one pinprick type filament probe;
   wherein the filament module comprises a rotating reel module having a plurality of compartments, wherein each of the probes is located in one of said compartments.
Clause 22. The device according to clause 21, wherein the rotating reel module further comprises an opening wherein rotation of the reel module aligns one of the probes with said opening thereby allowing output of the aligned probe.
Clause 23. The device according to clauses 21 or 22, wherein the rotating reel module further comprises a mechanism for rotating the reel.
Clause 24. The device according to any of clauses 21 to 23, the filament module further comprising a probe actuator.
Clause 25. The device according to clause 24, wherein the probe actuator comprises a piston module having a piston and a cylinder, the piston configured to be displaceable towards the rotating reel module.
Clause 26. The device according to clause 25, wherein the piston comprises a proximal end configured to be slidable in the cylinder and a distal end configured to be coupled to a coupling element of an aligned probe.
Clause 27. The device according to clause 28, wherein the distal end comprises a fastening element and the coupling element comprises a corresponding fastening element.
Clause 28. The device according to any of clauses 21 to 27, further comprising at least one arm, having a proximal end coupled to the main unit and a distal end coupled to the filament module.
Clause 29. The device according to clause 28, further comprising at least a thermovibrating module coupled to a second distal end of the at least one arm, said thermovibrating module comprising at least a vibrating unit.
Clause 30. The device according to clause 29, wherein the at least one extensible arm comprises a first arm and a second arm, wherein the first arm is coupled to the filament module and the second arm is coupled to the thermovibrating module.
Clause 31. The device according to clause 29 or 30, wherein each arm is extensible and comprises a plurality of joints, configured to attribute a plurality of degrees of freedom of movement to the filament and thermovibrating modules thereby allowing placement of each of the filament and thermovibrating modules at any part of the body without the need to reposition the body.
Clause 32. The device according to clause 31, wherein each joint is independently controllable by the main unit.
Clause 33. The device according to any of clauses 29 to 32, wherein the thermovibrating unit comprises a piston module having a piston and a cylinder, the piston having a proximal end configured to be slideable in the cylinder.
Clause 34. The device according to clause 33, wherein the distal end of the piston comprises a heatable surface.
Clause 35. The device according to any of clauses 21 to 34, further comprising at least one positioning module coupled at least to the filament or thermovibrating module.
Clause 36. The device according to clause 35, wherein the positioning module comprises an opening allowing said probe to pass through the positioning module and contact the part of the body at a position indicated by said positioning module.
Clause 37. The device according to clause 36, wherein the positioning module comprises a light emitting module.
Clause 38. The device according to clause 37, wherein the light emitting module comprises a first light pointer emitting a first light beam and a second light pointer emitting a second light beam, wherein the pointer beams are configured to coincide at the contact position.
Clause 39. The device according to any of clauses 35 to 38, wherein the positioning module further comprises one or more piezoelectric transducers configured to obtain distance and direction information from the surface of the part of the body.
Clause 40. A device for performing a plurality of Quantitative Sensory Testing (QST) tests to a part of an unmoving body, the device comprising:
   a main unit;
   at least a thermovibrating module, coupled to the main unit, comprising at least a piston module having a piston and a cylinder, the piston having a proximal end configured to be slidable in the cylinder.
Clause 41. The device according to clause 42, wherein the distal end of the piston comprises a heatable surface.
Clause 42. The device according to any of clauses 40 to 41, further comprising at least one arm, having a proximal end coupled to the main unit and a distal end coupled to the thermovibrating module.
Clause 43. The device according to clause 44, further comprising at least a filament module coupled to a second distal end of the at least one arm.
Clause 44. The device according to clause 43, wherein the filament module comprises a plurality of von Frey type filament probes and at least one pinprick type filament probe.
Clause 45. The device according to clause 43 or 44, wherein the at least one arm comprises a first arm and a second arm, wherein the first arm is coupled to the filament module and the second arm is coupled to the thermovibrating module.
Clause 46. The device according to any of clauses 43 to 45, wherein each arm is extensible and comprises a plurality of joints, configured to attribute a plurality of degrees of freedom of movement to the filament or thermovibrating modules thereby allowing placement of each of the filament and thermovibrating modules at any part of the body without the need to reposition the body.
Clause 47. The device according to clause 46, wherein each joint is independently controllable by the main unit.
Clause 48. The device according to any of clauses 43 to 47, wherein the filament module comprises a rotating reel module having a plurality of compartments, wherein each of the probes is located in one of said compartments.
Clause 49. The device according to clause 48, wherein the rotating reel module further comprises an opening wherein rotation of the reel module aligns one of the probes with said opening thereby allowing output of the aligned probe.
Clause 50. The device according to clauses 48 or 49, wherein the rotating reel module further comprises a mechanism for rotating the reel.
Clause 51. The device according to any of clauses 48 to 50, the filament module further comprising a probe actuator.
Clause 52. The device according to clause 51, wherein the probe actuator comprises a piston module having a piston and a cylinder, the piston configured to be displaceable towards the rotating reel module.
Clause 53. The device according to clause 52, wherein the piston comprises a proximal end configured to be slidable in the cylinder and a distal end configured to be coupled to a coupling element of an aligned probe.
Clause 54. The device according to clause 53, wherein the distal end comprises a fastening element and the coupling element comprises a corresponding fastening element.
Clause 55. The device according to any of clauses 40 to 54, further comprising at least one positioning module coupled at least to the filament or to the thermovibrating module.
Clause 56. The device according to clause 55, wherein the positioning module comprises an opening allowing said probe to pass through the positioning module and contact the part of the body at a position indicated by said positioning module.
Clause 57. The device according to clause 56, wherein the positioning module comprises a light emitting module.
Clause 58. The device according to clause 57, wherein the light emitting module comprises a first light pointer emitting a first light beam and a second light pointer emitting a second light beam, wherein the pointer beams are configured to coincide at the contact position.
Clause 59. The device according to any of clauses 55 to 58, wherein the positioning module further comprises one or more piezoelectric transducers configured to obtain distance and direction information from the surface of the part of the body.
Clause 60. The device according to any of previous clauses, wherein the main unit comprises a controller configured to control one or more of the filament module, the thermovibrating module and the at least one extensible arm.
Clause 61. The device according to any of previous clauses, wherein the main unit comprises a user interface module.
Clause 62. The device according to clause 61, wherein the user interface module comprises a display.
Clause 63. The device according to clause 62, wherein the display is a touch sensitive display.
Clause 64. The device according to any of clauses 61 to 63, wherein the user interface module further comprises a keyboard.
Clause 65. The device according to any of previous clauses, further comprising an input device configured to be used by the patient to indicate a perception of a test.
Clause 66. The device according to clause 65, wherein the input device comprises at least one button and the user indicates the perception of a test by pressing the button.
Clause 67. The device according to clause 66, wherein the input device comprises at least one ergonomic joystick.
Clause 68. The device according to any of previous clauses, further comprising an audio and/or visual stimulating device to alert the patent about the beginning of a test.
Clause 69. The device according to clause 68, wherein the main unit further comprises a test simulation module, configured to perform erroneous tests or to actuate the audio and/or visual stimulating device.
Clause 70. A programmable controller configured to control a device according to any of clauses 1 to 69.
Clause 71. A computer program product comprising instructions to provoke that programmable controller controls a device according to any of clauses 1 to 69.
Clause 72. A computer program product according to clause 71, stored in recording media.
Clause 73. A computer program product according to clause 71, carried by a carrier signal.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

## Claims

1. A device for performing a plurality of Quantitative Sensory Testing (QST) tests, the device comprising:
a main unit;
at least one extensible arm, having a proximal end coupled to the main unit;
at least one filament module coupled to a first distal end of the at least one arm, said filament module comprising a plurality of von Frey type filament probes and at least one pinprick type filament probe;
at least one thermovibrating module coupled to a second distal end of the at least one arm.

2. The device according to claim 1, wherein the at least one extensible arm comprises a first extensible arm and a second extensible arm, wherein the first extensible arm is coupled to the filament module and the second extensible arm is coupled to the thermovibrating module.

3. The device according to claim 1 or 2, wherein each arm comprises a plurality of joints, configured to attribute a plurality of degrees of freedom of movement to the filament and/or to the thermovibrating modules thereby allowing placement of each of the filament and thermovibrating modules at any part of the body without the need to reposition the body.

4. The device according to claim 3, wherein each joint is independently controllable by the main unit.

5. The device according to any of the above claims wherein the filament module comprises a rotating reel module having a plurality of compartments, wherein each of the probes is located in one of said compartments.

6. The device according to claim 5, wherein the rotating reel module further comprises an opening wherein rotation of the reel aligns one of the probes with said opening thereby allowing output of the aligned probe.

7. The device according to claims 5 or 6, wherein the rotating reel module further comprises a mechanism for rotating the reel.

8. The device according to any of claims 5 to 7, wherein the filament module further comprises a fastening element for coupling to said at least one arm.

9. The device according to any of claims 5 to 8, the filament module further comprising a probe actuator.

10. The device according to any of the previous claims, wherein the probe actuator comprises a piston module having a piston and a cylinder, the piston configured to be displaceable towards the rotating reel module.

11. The device according to claim 10, wherein the piston comprises a proximal end configured to be slidable in the cylinder and a distal end configured to be coupled to a coupling element of a probe.

12. The device according to claim 11, wherein the distal end comprises a fastening element and the coupling element comprises a corresponding fastening element.

13. The device according to any of previous claims, wherein the thermovibrating module comprises a piston module having a piston and a cylinder, the piston having a proximal end configured to be slidable in the cylinder.

14. The device according to claim 13, wherein a distal end of the piston comprises a heatable surface.

15. The device according to any of claims 13 to 14, wherein the thermovibrating module further comprises a fastening element for coupling to said at least one arm.
